# EUROPEAN PATENT APPLICATION

(11) **EP 2 716 251 A1**
(43) Date of publication of application: **09.04.2014**
(21) Application number: 12807768.2
(22) Date of filing: 22.05.2012
(51) Int. Cl.: A61B 18/18

(54) **BIPOLAR NEEDLE-SHAPED MICROWAVE SURGICAL INSTRUMENT**

(30) Priority: 07.07.2011 JP 2011151290
(71) Applicant: Yamashina Seiki Co., Ltd., Ritto-shi Shiga 520-3001 (JP)
(72) Inventor: HOSAKA, Makoto, Ritto-shi Shiga 520-3001 (JP); ABE, Kazuo, Ritto-shi Shiga 520-3001 (JP); UEMURA, Eiichi, Ritto-shi Shiga 520-3001 (JP)
(74) Representative: Reboussin, Yohann Mickaël Noël
(86) International application number: PCT/JP2012/062993
(87) International publication number: WO 2013/005484

(57) **Abstract**

An object of the present invention is to provide a microwave operation device which reliably and easily enables coagulation and the like on the surface of a biological tissue, and allows sufficient coagulation and the like in the vicinity of the end of the microwave operation device when the microwave operation device is inserted into the biological tissue to coagulate its deep portion and do the like.

The present invention provides a bipolar needle type microwave operation device, wherein at least one pair of rod-like electrodes are parallel to a center axis of the electrodes in their entirety, an insulator being filled between the paired electrodes. Preferably, at least two pairs of electrodes are alternate so that the electrodes having the same polarity are not adjacent to each other, and the ends of the paired electrodes having the same polarity are bridged with the same material as the electrodes. Preferably, a perpendicular distance between each of the electrodes and the center axis is within 2 mm.

## Description

### TECHNICAL FIELD

The present invention relates to a bipolar needle type microwave operation device.

### BACKGROUND ART

Conventionally, a bipolar needle type microwave operation device including an operation electrode including a tubular ground electrode inserted into a biological tissue, a tubular insulator provided in the core of the ground electrode and having an end extended from the end of the ground electrode, and a rod-like center electrode provided in the core of the insulator, having a microwave emission portion at the end thereof extended from the end of the insulator, and inserted into the biological tissue together with the end of the insulator, wherein the center electrode includes a rod-like center body and a center outer layer body on the outer periphery of the center body, wherein the center outer layer body and the ground electrode are made of a metal having a lower electric resistivity than the center body, wherein the center body is made of a metal having a higher hardness than the center outer layer body has been known (Patent document 1).

### PRIOR ART DOCUMENTS

### PATENT DOCUMENTS

Patent document 1: JP-A-10-137258

### SUMMARY OF THE INVENTION

### PROBLEMS TO BE SOLVED BY THE INVENTION

However, in the conventional microwave operation device, the pair of electrodes cannot be closely contacted onto the biological tissue at the same time. Consequently, coagulation and the like (coagulation, stop of bleeding, and cauterization) on the surface of the biological tissue are substantially impossible. In addition, coagulation and the like in the vicinity of the end of the microwave operation device is insufficient when the microwave operation device is inserted into the biological tissue to coagulate its deep portion and do the like.

That is, an object of the present invention is to provide a microwave operation device which reliably and easily enables coagulation and the like on the surface of a biological tissue, and allows sufficient coagulation and the like in the vicinity of the end of the microwave operation device when the microwave operation device is inserted into the biological tissue to coagulate its deep portion and do the like.

### SOLUTIONS TO THE PROBLEMS

In a bipolar needle type microwave operation device of the present invention, at least one pair of rod-like electrodes are parallel to a center axis of the electrodes in their entirety, an insulator being filled between the paired electrodes.

The at least one pair of rod-like electrodes may include at least one pair of rod-like electrodes (at least two rod-like electrodes), and may include two pairs or more of rod-like electrodes (four or more rod-like electrodes). Preferably, the at least one pair of rod-like electrodes include one pair to five pairs of rod-like electrodes. More preferably, the at least one pair of rod-like electrodes include one pair to three pairs of rod-like electrodes. Particularly preferably, the at least one pair of rod-like electrodes include one pair or two pairs of rod-like electrodes (two or four rod-like electrodes) .

In the at least one pair of rod-like electrodes, distances between two electrode surfaces crossing a straight line which passes through the center axis and is perpendicular to the center axis and the center axis are not always the same, and the electrode surfaces may be formed on surfaces having the same center axis (concentric axis) (on curved surfaces or planes) . That is, the rod-like electrodes may be arranged on the side surfaces of a circular cylinder, an elliptic cylinder, a polygonal cylinder, a regular polygonal cylinder and the like. Each of the electrode surfaces does not include the surface contacted onto the insulator, and is a surface which functions as an electrode, that is, a surface which can come into contact with a biological tissue. In addition, the term "rod-like" may include a shape in which a length in the center axis direction is longer than a length perpendicular to the center axis, and its cross-sectional shape is not particularly limited.

The center axis is the center axis (concentric axis) of the electrodes in their entirety including the at least one pair of rod-like electrodes.

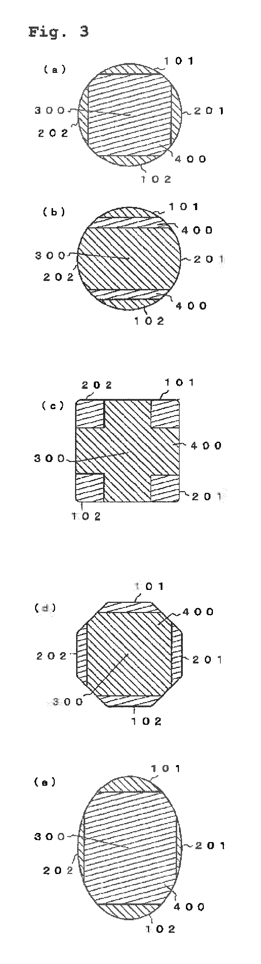

The at least one pair of rod-like electrodes may independently function as electrodes. Among two pairs or more of rod-like electrodes, the paired electrodes having the same polarity may be integrated with each other, and only the electrode surfaces may function independently {see Figs. 3 (b), 4(b), 4(c), 5(b), 5(c), 6, 12 - 13, and 14 - 15).

The bipolar needle type microwave operation device of the present invention may include, in addition to the at least one pair of rod-like electrodes, at least one pair of sectionally split electrodes (at least two sectionally split electrodes) arranged along the same center axis.

In such one pair of sectionally split electrodes, electrodes formed by sectionally splitting a circular cylindrical shape, a regular polygonal cylindrical shape, an elliptic cylindrical shape, a spherical shape, or an ellipsoidal body shape may be arranged along the center axis.

The end of the bipolar needle type microwave operation device of the present invention may be planar, spherical, sharp, and rounded although it is sharp.

The insulator is filled between the paired electrodes, and is not limited as long as it is an electric insulator. Examples of the preferred electric insulator include engineering plastics (polyetheretherketone (PEEK), polyethersulfone (PES) , polyamide (PA), polyamide imide (PAI), polyimide (PI), polyphenylene sulfide (PPS), polybenzimidazole (PBI) and the like}, fluororesins

(polytetrafluoroethylene, polychlorotrifluoroethylene, polyvinylidene fluoride, polyvinyl fluoride, a perfluoroalkoxy fluororesin, a tetrafluoroethylene-hexafluoropropylene copolymer, an ethylene-tetrafluoroethylene copolymer, an ethylene-chlorotrifluoroethylene copolymer and the like}, and ceramics {alumina (Al₂O₃), zirconia (ZrO₂), silicon carbide (SiC), silicon nitride (Si₃N₄) and the like}.

The at least one pair of rod-like electrodes will be described more specifically with reference to the drawings.

Referring to the following drawings, each of rod-like electrodes (100s) and each of rod-like electrodes (200s) are paired, and each of sectionally split electrodes (800s) and each of sectionally split electrodes (900s) are paired.

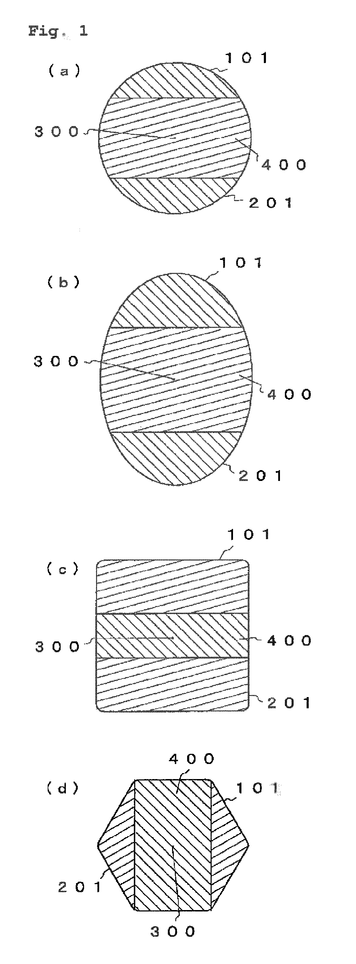

Figs. 1(a) to 2(d) are cross-sectional views of electrode portions of the bipolar needle type microwave operation device of the present invention in which one pair of rod-like electrodes are arranged, the cross sections thereof perpendicular to the center axis being schematically shown.

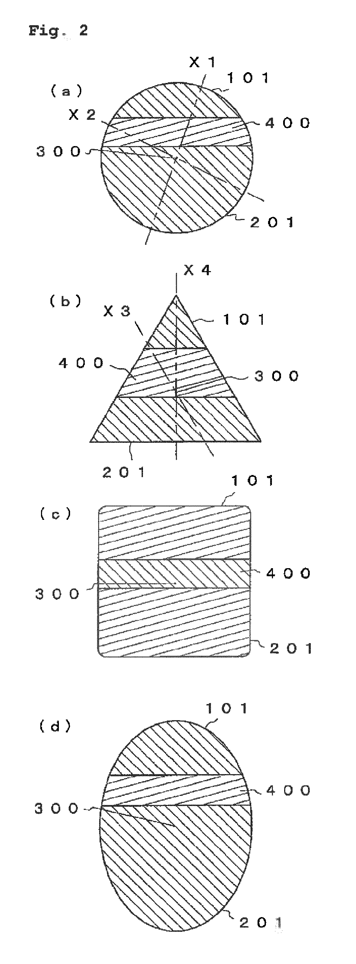

Fig. 1(a) is a cross-sectional view which conceptually shows an example in which one pair of rod-like electrodes (two rod-like electrodes; 101, 201) sandwich a rod-like insulator (400) therebetween to form a circular cylindrical shape. Fig. 1(b) is a cross-sectional view which conceptually shows an example in which an elliptic cylindrical shape is formed in the same manner. Fig. 1(c) is a cross-sectional view which conceptually shows an example in which a regular square cylindrical shape is formed in the same manner. Fig. 1(d) is a cross-sectional view which conceptually shows an example in which a regular hexagonal cylindrical shape is formed in the same manner. In Figs. 1(a) to 1(d), distances between two electrode surfaces crossing a straight line which passes through a center axis (300) and is perpendicular to the center axis (300) and the center axis (300) are the same, but the distances are not always the same for all the electrode surfaces (see Figs. 2 (a) to 2(d)).

Fig. 2(a) is a cross-sectional view which conceptually shows an example in which one pair of rod-like electrodes (two rod-like electrodes; 101, 201) sandwich a rod-like insulator (400) therebetween to form a circular cylindrical shape. In Fig. 2(a), distances between two electrode surfaces crossing a straight line (x1) which passes through a center axis (300) and is perpendicular to the center axis (300) and the center axis (300) are the same. Since there is only one electrode surface with respect to a straight line (x2), the distances cannot be the same. However, even such electrodes configure the bipolar needle type microwave operation device of the present invention.

Fig. 2(b) is a cross-sectional view which conceptually shows an example in which a triangular cylindrical shape is formed as in Fig. 2(a). In Fig. 2(b), distances between two electrode surfaces crossing a straight line (x3) which passes through the center axis (300) and is perpendicular to the center axis (300) and the center axis (300) are the same. The distances cannot be the same for another straight line (e.g., x4). However, even such electrodes configure the bipolar needle type microwave operation device of the present invention.

Fig. 2(c) is a cross-sectional view which conceptually shows an example in which a regular square cylindrical shape is formed as in Fig. 2 (a). Fig. 2 (d) is a cross-sectional view which conceptually shows an example in which an elliptic cylindrical shape is formed in the same manner.

Figs. 3 (a) to 3 (e) are cross-sectional views of electrode portions of the bipolar needle type microwave operation device of the present invention in which two pairs of rod-like electrodes are arranged, the cross sections thereof perpendicular to the center axis being schematically shown.

Fig. 3(a) is a cross-sectional view which conceptually shows an example in which two pairs of rod-like electrodes (four rod-like electrodes; 101, 102, 201, 202) sandwich a rod-like insulator (400) therebetween to form a circular cylindrical shape. Fig. 3(b) is a cross-sectional view which conceptually shows an example in which two pairs of rod-like electrodes include three rod-like electrodes (101, 102, 201 - 202) so that these sandwich the rod-like insulator (400) therebetween to form a circular cylindrical shape. The electrodes (201) and (202) are integrated with each other.

Fig. 3(c) is a cross-sectional view which conceptually shows an example in which a regular square cylindrical shape is formed as in Fig. 3 (a). Fig. 3 (d) is a cross-sectional view which conceptually shows an example in which a regular octagonal cylindrical shape is formed in the same manner. Fig. 3(e) is a cross-sectional view which conceptually shows an example in which an elliptic cylindrical shape is formed in the same manner.

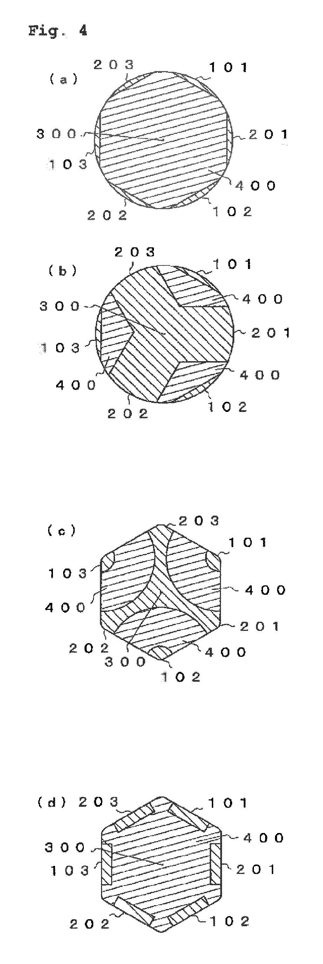

Figs. 4 (a) to 4 (d) are cross-sectional views of electrode portions of the bipolar needle type microwave operation device of the present invention in which three pairs of rod-like electrodes are arranged, the cross sections thereof perpendicular to the center axis being schematically shown.

Fig. 4(a) is a cross-sectional view which conceptually shows an example in which three pairs of rod-like electrodes (six rod-like electrodes; 101, 102, 103, 201, 202, 203) sandwich a rod-like insulator (400) therebetween to form a circular cylindrical shape. Fig. 4(b) is a cross-sectional view which conceptually shows an example in which three pairs of electrodes include three rod-like electrodes (101, 102, 103) and a polygonal cylindrical electrode (201 - 202 - 203) whose cross section perpendicular to the center axis is three-forked so that these sandwich the rod-like insulator (400) to form a circular cylindrical shape. The electrodes (201), (202), and (203) are integrated with each other. Fig. 4 (c) is a cross-sectional view which conceptually shows an example in which a regular hexagonal cylindrical shape is formed as in Fig. 4 (b). In Fig. 4 (c), the electrodes (201), (202), and (203) are integrated with each other. Fig. 4(d) is a cross-sectional view which conceptually shows an example in which a regular hexagonal cylindrical shape is formed as in Fig. 4(a).

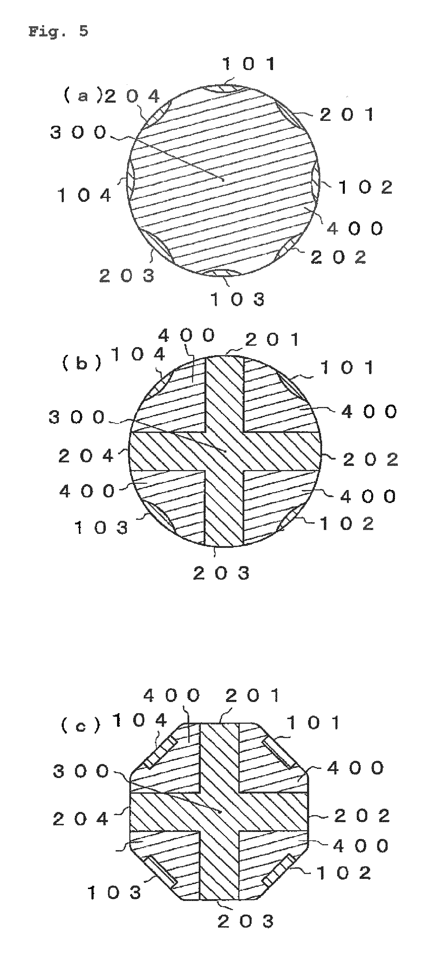

Figs. 5 (a) to 5 (c) are cross-sectional views of electrode portions of the bipolar needle type microwave operation device of the present invention in which four pairs of rod-like electrodes are arranged, the cross sections thereof perpendicular to the center axis being schematically shown.

Fig. 5(a) is a cross-sectional view which conceptually shows an example in which four pairs of rod-like electrodes (eight rod-like electrodes; 101, 102, 103, 104, 201, 202, 203, 204) sandwich a rod-like insulator (400) therebetween to form a circular cylindrical shape. Fig. 5(b) is a cross-sectional view which conceptually shows an example in which four pairs of electrodes include four rod-like electrodes (101, 102, 103, 104) and a polygonal cylindrical electrode (201 - 202 - 203 - 204) whose cross section perpendicular to the center axis is cross-shaped so that these sandwich the rod-like insulator (400) therebetween to form a circular cylindrical shape. The electrodes (201), (202), (203), and (204) are integrated with each other. Fig. 5(c) is a cross-sectional view which conceptually shows an example in which a regular octagonal cylindrical shape is formed as in 54(b). In Fig. 5(c), the electrodes (201), (202), (203), and (204) are integrated with each other.

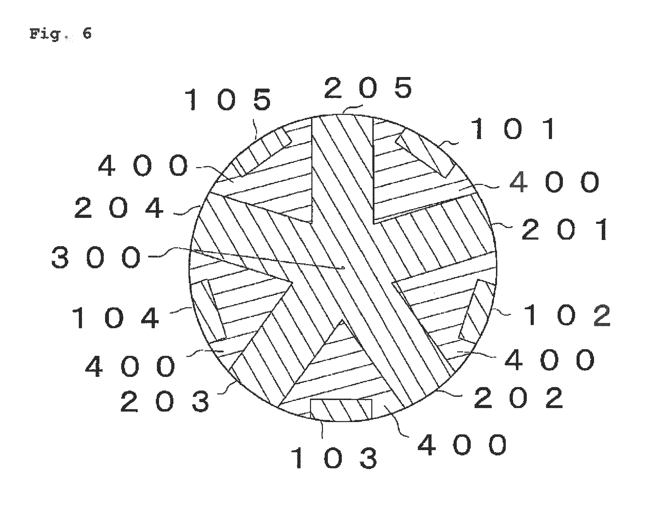

Fig. 6 is a cross-sectional view of electrode portions of the bipolar needle type microwave operation device of the present invention in which five pairs of rod-like electrodes are arranged, the cross section thereof perpendicular to the center axis being schematically shown. Fig. 6 is a cross-sectional view which conceptually shows an example in which five pairs of electrodes include five rod-like electrodes (101, 102, 103, 104, 105) and a polygonal cylindrical electrode (201 - 202 - 203 - 204 - 205) whose cross section perpendicular to the center axis is five-forked so that these sandwich a rod-like insulator (400) therebetween to form a circular cylindrical shape. The electrodes (201), (202), (203), (204), and (205) are integrated with each other.

Next, the at least one pair of sectionally split electrodes which may be added to the at least one pair of rod-like electrodes will be described more specifically with reference to the drawings.

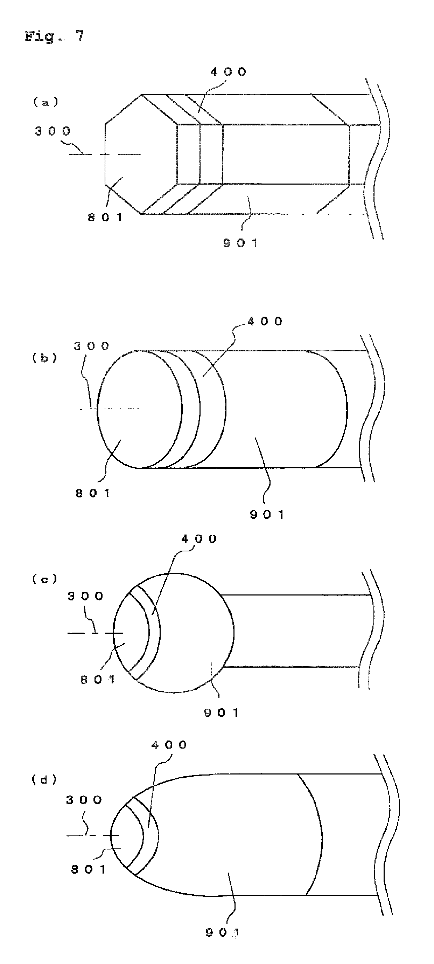

Figs. 7(a) to 7(d) are perspective views schematically showing one pair of sectionally split electrodes.

Fig. 7 (a) is a perspective view which conceptually shows an example in which one pair of hexagonal cylindrical-shaped sectionally split electrodes (two electrodes; 801, 901) sandwich a hexagonal cylindrical-shaped sectionally split insulator (400) therebetween to form a hexagonal cylindrical shape. Fig. 7 (b) is a perspective view which conceptually shows an example in which a circular cylindrical shape is formed in the same manner. Fig. 7(c) is a perspective view which conceptually shows an example in which a spherical shape is formed in the same manner. Fig. 7 (d) is a perspective view which conceptually shows an example in which an ellipsoidal body shape (spindle shape) is formed in the same manner.

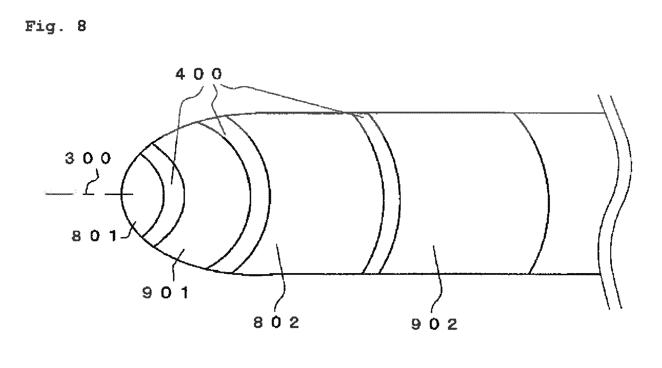

Fig. 8 is a perspective view schematically showing two pairs of sectionally split electrodes. That is, Fig. 8 is a perspective view which conceptually shows an example in which two pairs of electrodes (four electrodes; 801, 802, 901, 902) formed by sectionally splitting an ellipsoidal body shape (spindle shape) sandwich an insulator (400) therebetween along the same center axis.

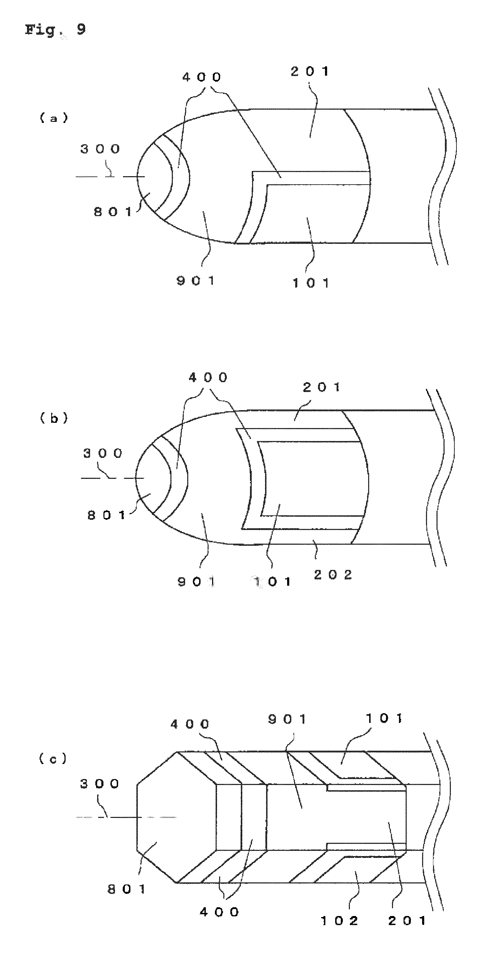

Figs. 9(a) to 9(c) are perspective views of electrode portions of the bipolar needle type microwave operation device of the present invention which includes the at least one pair of sectionally split electrodes in addition to the at least one pair of rod-like electrodes.

Fig. 9 (a) is a perspective view which conceptually shows an example in which one pair of sectionally split electrodes (two electrodes; 801, 901) formed by sectionally splitting an ellipsoidal body shape (spindle shape) sandwich an insulator (400) therebetween along the same center axis, and one pair of rod-like electrodes (two electrodes; 101, 201) sandwich the insulator (400) therebetween. That is, Fig. 9(a) is a perspective view which conceptually shows an example in which the electrode portion shown in Fig. 7(d) and the electrode portion shown in Fig. 1(a) are provided.

Fig. 9(b) is a perspective view which conceptually shows an example in which one pair of sectionally split electrodes (two electrodes; 801, 901) formed by sectionally splitting an ellipsoidal body shape (spindle shape) sandwich the insulator

(400) therebetween along the same center axis, and two pairs of rod-like electrodes (four electrodes; 101, 102, 201, 202) sandwich the insulator (400) therebetween. That is, Fig. 9 (b) is a perspective view which conceptually shows an example in which the electrode portion shown in Fig. 7 (d) and the electrode portion shown in Fig. 3(a) are provided.

Fig. 9(c) is a perspective view which conceptually shows an example in which one pair of sectionally split electrodes (two electrodes; 801, 901) formed by sectionally splitting a hexagonal cylindrical shape sandwich the insulator (400) therebetween along the same center axis, and three pairs of rod-like electrodes (six electrodes; 101, 102, 103, 201, 202, 203) sandwich the insulator (400) therebetween. That is, Fig. 9(c) is a perspective view which conceptually shows an example in which the electrode portion shown in Fig. 7(a) and the electrode portion shown in Fig. 4(d) are provided.

The at least one pair of rod-like electrodes may be made of metals or ceramics as long as they function as electrodes, and may be surface protected (metal plated or coated with the fluororesin and the like) (this is ditto for the at least one pair of sectionally split electrodes). In addition, preferably, among the at least one pair of rod-like electrodes, the length of the electrode connected to the center electric conductor of the coaxial cable is natural number times (nλ) a wavelength (λ) of a microwave passing through the biological tissue or is a fraction of a natural number (λ/n) thereof (n is a natural number) (this is ditto for the at least one pair of sectionally split electrodes). The length of the electrode connected to the external electric conductor of the coaxial cable is not particularly limited. In addition, the electrode (201) in Fig. 11, the electrodes (201) and (202) in Figs. 12 and 13, and the electrodes (201) and (202) in Figs. 14 and 15 correspond to the electrode connected to the center electric conductor of the coaxial cable with reference to the drawings shown in "MODE FOR CARRYING OUT THE INVENTION".

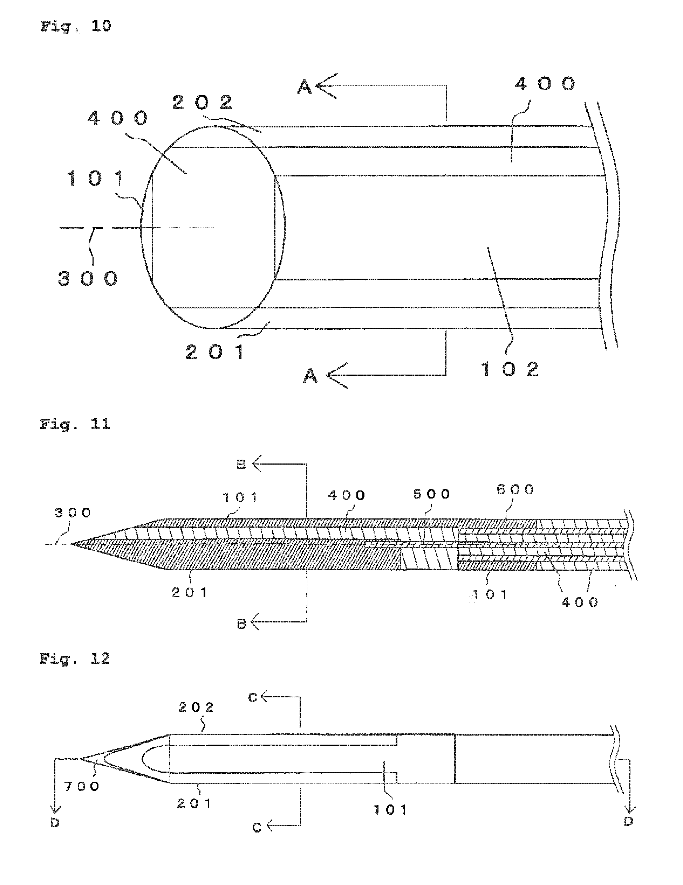

Preferably, the at least one pair of rod-like electrodes include at least two pairs of rod-like electrodes, the at least two pairs of rod-like electrodes are alternate so that the rod-like electrodes having the same polarity are not adjacent to each other. Preferably, the ends of the paired rod-like electrodes having the same polarity are bridged with the same material as the electrodes. That is, the ends of the electrodes including the sectionally split electrodes are bridged since they are already covered with the electrodes. On the other hand, as shown in Figs. 12 - 13 and Figs. 14 - 15, preferably, the electrodes not including the sectionally split electrodes are bridged.

A perpendicular distance between each of the rod-like electrodes and the center axis is preferably within 2 mm, more preferably, within 1.3 mm, particularly preferably, within 1 mm, and most preferably, within 0.9 mm. That is, for instance, the circle of the cross-section of the electrodes in Fig. 1(a) preferably has a diameter of 4 mm or less. The term "perpendicular distance" is the distance on the perpendicular line from the center axis (the distance between the center axis and each of the electrode surfaces).

The bipolar needle type microwave operation device of the present invention which is inserted into an endoscope or a catheter is preferable as an operation device.

The bipolar needle type microwave operation device of the present invention is preferably coated in its entirety with the fluororesin. The bipolar needle type microwave operation device of the present invention which is coated with the fluororesin is unlikely to be dirty, can be easily cleaned even when it becomes dirty, is hygienic, and can prevent heavy metal melting. Examples of the flororesin include known fluororesins {polytetrafluoroethylene, polychlorotrifluoroethylene, polyvinylidene fluoride, polyvinyl fluoride, a perfluoroalkoxy fluororesin, a tetrafluoroethylene-hexafluoropropylene copolymer, an ethylene-tetrafluoroethylene copolymer, and an ethylene-chlorotrifluoroethylene copolymer).

In the bipolar needle type microwave operation device of the present invention, the microwave can be applied between the at least one pair of rod-like electrodes (and between the at least one pair of sectionally split electrodes when the sectionally split electrodes are included). By applying the microwave, the biological tissue around the at least one pair of rod-like electrodes (and the biological tissue around the at least one pair of sectionally split electrodes when these are included) can be heated. Stop of bleeding, coagulation and the like can thus be achieved.

The microwave is preferably an electric wave having a frequency of 13 MHz to 25 GHz, more preferably, an electric wave having a frequency of 900 MHz to 6 GHz, particularly preferably, an electric wave having a frequency of 2.45 GHz.

A microwave operation apparatus of the present invention includes the bipolar needle type microwave operation device, a microwave transmitter, and the coaxial cable connecting the bipolar needle type microwave operation device and the microwave transmitter.

The bipolar needle type microwave operation device and the microwave transmitter are connected by the coaxial cable. The microwave generated by the microwave transmitter is transmitted to the at least one pair of electrodes via the coaxial cable.

The microwave transmitter is not limited as long as it can transmit the above frequencies, and its output is preferably approximately 10 W to 200 W.

### ADVANTAGES OF THE INVENTION

The microwave operation device of the present invention reliably and easily enables coagulation and the like on the surface of the biological tissue, and allows sufficient coagulation and the like in the vicinity of the end of the microwave operation device when the microwave operation device is inserted into the biological tissue to coagulate its deep portion and do the like.

In addition, the bipolar needle type microwave operation device of the present invention which can be reduced in diameter is applicable to an endoscopic operation and a celioscopic operation. The bipolar needle type microwave operation device of the present invention is also applicable to a typical direct vision operation (surgical operation, brain surgery, otological surgery and the like).

Further, the bipolar needle type microwave operation device of the present invention enables stop of bleeding and coagulation of a duct tissue (a blood vessel, a bile duct and the like), and stop of bleeding, coagulation and the like of a cancer tissue.

The microwave operation apparatus of the present invention includes the bipolar needle type microwave operation device, the microwave transmitter, and the coaxial cable. Therefore, the microwave operation apparatus of the present invention reliably and easily enables coagulation and the like on the surface of the biological tissue, and allows sufficient coagulation and the like in the vicinity of the end of the microwave operation device when the microwave operation device is inserted into the biological tissue to coagulate its deep portion and do the like.

In addition, the microwave operation apparatus of the present invention is applicable to an endoscopic operation and a celioscopic operation. The microwave operation apparatus of the present invention is also applicable to a typical direct vision operation (surgical operation, brain surgery, otological surgery and the like).

### BRIEF DESCRIPTION OF THE DRAWINGS

Figs. 1 (a) to 1 (d) are cross-sectional views of electrode portions of an embodiment of a bipolar needle type microwave operation device of the present invention {an example in which one pair of rod-like electrodes sandwich a rod-like insulator therebetween to form a circular cylindrical shape (a), an elliptic cylindrical shape (b), a regular square cylindrical shape (c), or a regular hexagonal cylindrical shape (d)}, the cross sections thereof perpendicular to a center axis being schematically shown.
Figs. 2 (a) to 2 (d) are cross-sectional views of electrode portions of an embodiment of a bipolar needle type microwave operation device of the present invention {an example in which one pair of rod-like electrodes sandwich a rod-like insulator therebetween to form a circular cylindrical shape (a), a triangular cylindrical shape (b), a regular square cylindrical shape (c), or an elliptic cylindrical shape (d)}, the cross sections thereof perpendicular to a center axis being schematically shown.
Figs. 3 (a) to 3 (e) are cross-sectional views of electrode portions of an embodiment of a bipolar needle type microwave operation device of the present invention {an example in which two pairs of rod-like electrodes sandwich a rod-like insulator therebetween to form a circular cylindrical shape (a), a regular square cylindrical shape (c), a regular octagonal cylindrical shape (d), or an elliptic cylindrical shape (e), and an example in which two pairs of electrodes include three rod-like electrodes so that these sandwich the rod-like insulator therebetween to form a circular cylindrical shape (b)}, the cross sections thereof perpendicular to a center axis being schematically shown.
Figs. 4 (a) to 4 (d) are cross-sectional views of electrode portions of an embodiment of a bipolar needle type microwave operation device of the present invention {an example in which three pairs of rod-like electrodes sandwich a rod-like insulator to form a circular cylindrical shape (a) or a regular hexagonal cylindrical shape (d), and an example in which three pairs of electrodes include three rod-like electrodes and a polygonal cylindrical electrode whose cross section perpendicular to a center axis is three-forked so that these sandwich the rod-like insulator therebetween to form a circular cylindrical shape (b) or a regular hexagonal cylindrical shape (c)}, the cross sections thereof perpendicular to the center axis being schematically shown.
Figs. 5 (a) to 5 (c) are cross-sectional views of electrode portions of an embodiment of a bipolar needle type microwave operation device of the present invention {an example in which four pairs of rod-like electrodes sandwich a rod-like insulator to form a circular cylindrical shape (a), and an example in which four pairs of electrodes include four rod-like electrodes and a polygonal cylindrical electrode whose cross section perpendicular to a center axis is cross-shaped so that these sandwich the rod-like insulator therebetween to form a circular cylindrical shape (b) or a regular octagonal cylindrical shape (c)}, the cross sections thereof perpendicular to the center axis being schematically shown.
Fig. 6 is a cross-sectional view of electrode portions of an embodiment of a bipolar needle type microwave operation device of the present invention {an example in which five pairs of electrodes include five rod-like electrodes and a polygonal cylindrical electrode whose cross section perpendicular to a center axis is five-forked (five radial lines) so that these sandwich a rod-like insulator therebetween to form a circular cylindrical shape}, the cross section thereof perpendicular to the center axis being schematically shown.
Figs. 7(a) to 7(d) are perspective views which schematically show one pair of sectionally split electrodes which can be included in a bipolar needle type microwave operation device of the present invention.
Fig. 8 is a perspective view which schematically shows two pairs of sectionally split electrodes which can be included in a bipolar needle type microwave operation device of the present invention.
Figs. 9(a) to 9(c) are perspective views which schematically show electrode portions of an embodiment of a bipolar needle type microwave operation device of the present invention {an example in which in addition to at least one pair of rod-like electrodes, at least one pair of sectionally split electrodes are included}.
Fig. 10 is a perspective view which schematically shows electrode portions of an embodiment of a bipolar needle type microwave operation device of the present invention {an example in which two pairs of rod-like electrodes are parallel to a center axis}.
Fig. 11 is a cross-sectional view which schematically shows electrode portions of an embodiment of a bipolar needle type microwave operation device of the present invention {an example in which one pair of rod-like electrodes are parallel to a center axis}.
Fig. 12 is a plan view which schematically shows electrode portions of an embodiment of a bipolar needle type microwave operation device of the present invention {an example in which two pairs of rod-like electrodes are parallel to a center axis, and the ends of the rod-like electrodes having the same polarity are bridged by a bridging portion with the same material as the rod-like electrodes}.

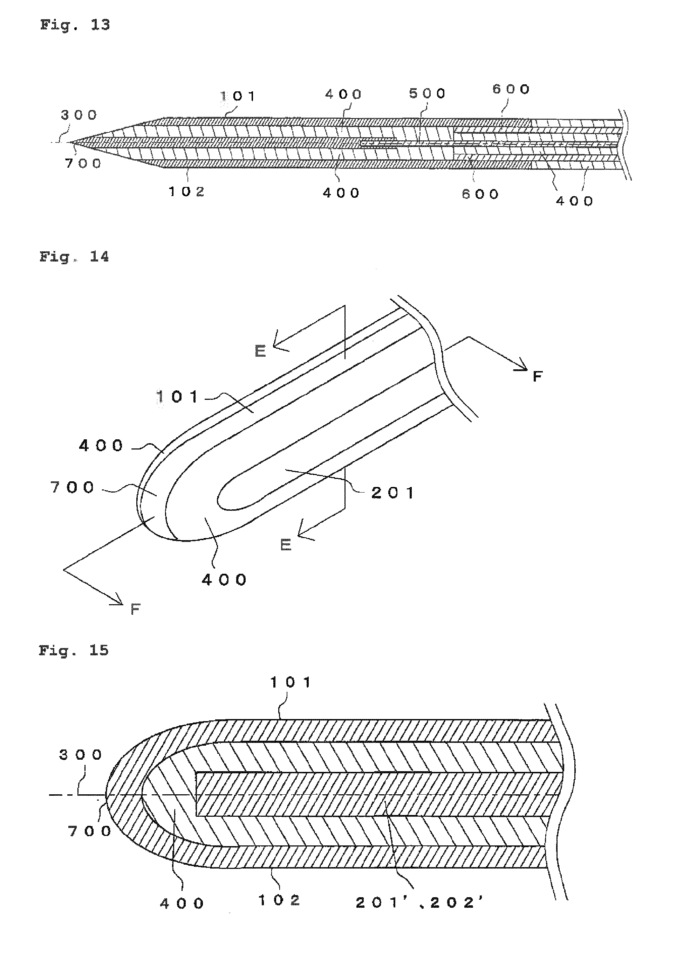

Fig. 13 is a cross-sectional view taken along line D - D in the plan view shown in Fig. 12.
Fig. 14 is a perspective view which schematically shows electrode portions of an embodiment of a bipolar needle type microwave operation device of the present invention {an example in which two pairs of electrodes include two rod-like electrodes so that these sandwich a rod-like insulator therebetween to form a circular cylindrical shape, and the ends of the rod-like electrodes having the same polarity are bridged by the bridging portion with the same material as the rod-like electrodes}.
Fig. 15 is a cross-sectional view taken along line F - F in the plan view shown in Fig. 14.

### MODE FOR CARRYING OUT THE INVENTION

Hereinafter, the bipolar needle type microwave operation device of the present invention will be described in more detail with reference to the drawings. Unless otherwise specified, the first described matter is sharably applicable to the description of the drawings thereafter.

### <Fig. 10>

Fig. 10 is a perspective view which schematically shows electrode portions of an embodiment of a bipolar needle type microwave operation device of the present invention.

In the bipolar needle type microwave operation device of the present invention shown in Fig. 10, two pairs of rod-like electrodes (four rod-like electrodes) are alternate to be symmetrical with respect to the same center axis and to be parallel to the center axis, and distances between two electrode surfaces crossing a straight line which passes through the center axis and is perpendicular to the center axis and the center axis are the same.

Fig. 3(a) shows electrode portions of the bipolar needle type microwave operation device shown in Fig. 10, the cross section thereof perpendicular to the center axis (cross section taken along line A - A) being schematically shown. That is, two pairs of rod-like electrodes (four electrodes 101, 102, 201, 202) sandwich a rod-like insulator (400) therebetween to form a circular cylindrical shape.

Two pairs of rod-like electrodes are connected to a center electric conductor (500) and an external electric conductor (600) of a coaxial cable (the coaxial cable is not shown in Fig. 10). The microwave is applied between two pairs of rod-like electrodes (101, 102, 201, 202) via the coaxial cable to heat a biological tissue around between two pairs of rod-like electrodes. Stop of bleeding, coagulation and the like can thus be achieved (the biological tissue around the side surfaces and end of the circular cylindrical shape can be efficiently heated).

The end of the bipolar needle type microwave operation device is planar in Fig. 10, but may be spherical, sharp, and rounded although it is sharp.

### <Fig. 11>

Fig. 11 is a cross-sectional view which schematically shows electrode portions of an embodiment of a bipolar needle type microwave operation device of the present invention.

In the bipolar needle type microwave operation device shown in Fig. 11, one pair of rod-like electrodes (two rod-like electrodes) are symmetrical with respect to the same center axis and are parallel to the center axis, and in the example in which distances between two electrode surfaces crossing a straight line which passes through the center axis and is perpendicular to the center axis and the center axis are the same, distances between two electrode surfaces crossing a straight line which passes through the center axis and is perpendicular to the center axis and the center axis are not always the same.

Fig. 2 (a) shows electrode portions of the bipolar needle type microwave operation device shown in Fig. 11, the cross section thereof perpendicular to the center axis (cross section taken along line B - B) being schematically shown. That is, one pair of rod-like electrodes (two rod-like electrodes 101, 201) sandwich a rod-like insulator (400) therebetween to form a circular cylindrical shape.

One pair of rod-like electrodes is connected to a center electric conductor (500) and an external electric conductor (600) of a coaxial cable. The microwave is applied between one pair of rod-like electrodes (101, 201) via the coaxial cable to heat a biological tissue around between one pair of rod-like electrodes (around the side surfaces and ends of the electrodes). Stop of bleeding, coagulation and the like can thus be achieved.

The end of the bipolar needle type microwave operation device is sharp in Fig. 11, but may be rounded, planar, and spherical.

### <Figs. 12 and 13>

Fig. 12 is a plan view which schematically shows electrode portions of an embodiment of a bipolar needle type microwave operation device of the present invention. Fig. 13 is a cross-sectional view taken along line D - D in the plan view shown in Fig. 12.

In the bipolar needle type microwave operation device of the present invention shown in Figs. 12 and 13, two pairs of rod-like electrodes (four rod-like electrodes) are alternate to be symmetrical with respect to the same center axis and to be parallel to the center axis, and distances between two electrode surfaces crossing a straight line which passes through the center axis and is perpendicular to the center axis and the center axis are the same.

Fig. 3 (b) shows electrode portions of the bipolar needle type microwave operation device shown in Figs. 12 and 13, the cross section thereof perpendicular to the center axis (cross section taken along line C - C) being schematically shown. That is, two pairs of electrodes include three rod-like electrodes (101, 102, 201 - 202) so that these sandwich a rod-like insulator

(400) therebetween to form a circular cylindrical shape. The rod-like electrodes (201) and (202) are integrated with each other. Among the rod-like electrodes (101, 102, 201 - 202) which are paired, the ends of the rod-like electrodes (201, 202) having the same polarity are bridged by a bridging portion (700) with the same material as the rod-like electrodes.

Two pairs of rod-like electrodes are connected to a center electric conductor (500) and an external electric conductor (600) of the coaxial cable. The microwave is applied between two pairs of rod-like electrodes (101, 102, 201 - 202) via the coaxial cable to heat a biological tissue around between two pairs of rod-like electrodes (around the side surfaces and ends of the electrodes) . Stop of bleeding, coagulation and the like can thus be achieved.

The end of the bipolar needle type microwave operation device is sharp in Figs. 12 and 13, but may be rounded, planar, and spherical.

### <Figs. 14 and 15>

Fig. 14 is a perspective view which schematically shows electrode portions of an embodiment of a bipolar needle type microwave operation device of the present invention. Fig. 15 is a cross-sectional view taken along line F - F in the plan view shown in Fig. 14.

In the bipolar needle type microwave operation device shown in Figs. 14 and 15, two pairs of rod-like electrodes (four rod-like electrodes) are alternate to be symmetrical with respect to the same center axis and to be parallel to the center axis, and distances between two electrode surfaces crossing a straight line which passes through the center axis and is perpendicular to the center axis and the center axis are the same.

Fig. 3(b) shows electrode portions of the bipolar needle type microwave operation device shown in Figs. 14 and 15, the cross section thereof perpendicular to the center axis (cross section taken along line E - E) being schematically shown. That is, two pairs of electrodes include two rod-like electrodes (101 - 102, 201 - 202) so that these sandwich a rod-like insulator

(400) therebetween to form a circular cylindrical shape. The rod-like electrodes (101) and (102) are integrated with each other to be U-shaped. The rod-like electrodes (201) and (202) are integrated with each other to be plate-shaped. Among the rod-like electrodes (101 - 102, 201 - 202) which are paired, the ends of the rod-like electrodes (101, 102) having the same polarity are bridged by a bridging portion (700) with the same material as the rod-like electrodes. In Fig. 15, the reference numerals 201' and 202' denote the cross sections of the members configuring the rod-like electrodes (201) and (202).

Two pairs of rod-like electrodes are connected to a center electric conductor (500) and an external electric conductor (600) of the coaxial cable. The microwave is applied between two pairs of rod-like electrodes (101 - 102, 201 - 202) via the coaxial cable to heat a biological tissue around between two pairs of rod-like electrodes (around the side surfaces and ends of the electrodes) . Stop of bleeding, coagulation and the like can thus be achieved.

The end of the bipolar needle type microwave operation device is spherical in Figs. 14 and 15, but may be planar, sharp, and rounded although it is sharp.

### DESCRIPTION OF REFERENCE SIGNS

- 101 to 105: Rod-like electrode
- 201 to 205: Rod-like electrode
- 300: Center axis
- 400: Insulator
- 500: Center electric conductor
- 600: External electric conductor
- 700: Bridging portion
- 801 to 802: Sectionally split electrode
- 901 to 902: Sectionally split electrode

## Claims

1. A bipolar needle type microwave operation device, wherein at least one pair of rod-like electrodes are parallel to a center axis of the electrodes in their entirety, an insulator being filled between the paired electrodes.

2. The bipolar needle type microwave operation device according to claim 1, wherein at least two pairs of electrodes are alternate so that the electrodes having the same polarity are not adjacent to each other, and the ends of the paired electrodes having the same polarity are bridged with the same material as the electrodes.

3. The bipolar needle type microwave operation device according to claim 1 or 2, wherein a perpendicular distance between each of the electrodes and the center axis is within 2 mm.

4. The bipolar needle type microwave operation device according to any one of claims 1 to 3, which is inserted into an endoscope or a catheter.

5. The bipolar needle type microwave operation device according to any one of claims 1 to 4, which is coated in its entirety with a fluororesin.

6. A microwave operation apparatus comprising:
the bipolar needle type microwave operation device according to any one of claims 1 to 5;
a microwave transmitter; and
a coaxial cable connecting the bipolar needle type microwave operation device and the microwave transmitter.
